# EUROPEAN PATENT SPECIFICATION

(11) **EP 0 591 091 B1**
(45) Date of publication and mention of the grant of the patent: **24.11.1999**
(21) Application number: 93610049.4
(22) Date of filing: 09.09.1993
(51) Int. Cl.: A61L 29/00, C08J 7/04, C09D 5/00

(54) **A method of producing an article with a coating having friction-reducing properties and coating composition**
Verfahren zur Herstellung eines Artikels mit Friktions-reduzierender Beschichtung und die Beschichtungszusammensetzung
Procédé pour produire un article avec revêtement ayant des propriétés anti-friction et la composition de revêtement

(30) Priority: 18.09.1992 DK 116092
(43) Date of publication of application: 06.04.1994
(73) Proprietor: MAERSK MEDICAL A/S, 3540 Lynge (DK)
(72) Inventor: Hansen, Charles Medom, DK-2970 Horsholm (DK); Just, Lisbeth Juhl, DK-2880 Bagsvaerd (DK)
(74) Representative: Zeuthen-Aagaard, Henrik

(56) References cited:
- GB-A- 2 064 556
- GB-A- 2 190 387
- US-A- 4 055 682
- US-A- 5 041 100

## Description

The present invention relates to a method of producing an article with a coating having friction-reducing properties in wet condition, said coating comprising a binder (a), a hydrophilic polymer (b) which is different from the binder (a), as well as, if desired, an osmolality-increasing compound (c), as well as to a coating material to be used by the method.

In the present description and claims the terms "binder" and "hydrophilic polymer" relate to two types of materials which are to be distinguished from each other.

The invention relates in particular, but not exclusively to articles of the above type to be used in connection with surgery, therapy, or diagnostic methods, such as articles to be inserted in a body cavity, such as catheters, guide wires, wound drains and fibre-optical articles. These articles can be made of plastics, such as PVC or polyurethane, or of metal or glass.

Several examples of these articles are known, such as catheters having a friction-reducing coating. In connection with catheters, the presence of the friction-reducing coating causes the surface of the catheter to be slippery and lubricating when said catheter is immersed into an aqueous, optionally saline solution prior to the insertion into a body cavity or when the catheter is contacted with an aqueous body liquid at the insertion into the body cavity.

Thus the discomfort experienced by the patient when the catheter is inserted into and removed from the body cavity is considerably reduced. The risk of damaging sensitive tissue in connection with the use of the catheter is at the same time considerably reduced.

The desired lubricating effect can be obtained by way of sustained release of hydrophilic polymer. When it is an article to be inserted into the human body and which must be removable after a long period of insertion in said human body, it is of vital importance that a sufficient amount of hydrophilic polymer is released whenever a lubricating effect is desired, i.e. during both the insertion and the removal thereof later on.

The production of hydrophilic coatings is encumbered with the problem that the coating must contain a considerable amount of hydrophilic polymer in order to possess a sufficient hydrophility, and the hydrophilic polymers are difficult to maintain on the surface of solid articles, especially when said articles are made of hydrophobic materials, such as PVC or polyurethane. Therefore it is usually necessary to use a binder in form of a hydrophobic material, said binder, however, reducing the hydrophilic character of the surface as well as its capacity of turning slippery and lubricating in wet condition.

A list of the prior art concerning hydrophilic lubricating, i.e. friction-reducing, coatings for medical application is found in a paper by Y.L. Fan "HYDROPHILIC LUBRICIOUS COATINGS FOR MEDICAL APPLICATIONS" from "POLYMERIC MATERIALS SCIENCE AND ENGINEERING"; Proceedings of the ACS Division of Polymeric Materials: Science and Engineering, vol. 63, autum meeting 1990, Washington, D.C. published in 1990 by AMERICAN CHEMICAL SOCIETY.

In US-PS No. 5,005,287 corresponding to EP-PS No. 289,996, Ritter suggests a method of applying a surface coating being slippery in wet condition onto the sides of a razor unit sliding against the skin during a wet shaving. The coating used is a solution containing a water-soluble polymer, especially poly-N-vinyl pyrrolidone (PVP), together with a radical-polymerisable vinyl monomer and a photoinitiator decomposed by way of radiation into free radicals.

As vinyl momoners Ritter uses according to the Examples tetra-hydrofuranyl-2-methylacrylate or a combination of N-vinyl pyrrolidone and trimethylolpropane monoacrylate, which are all relatively low molecular weight compounds. Usually such low molecular weight monomers are undesired in connection with materials to be in contact with the human body for a long period of time as such compounds are often injurious to health. In connection with coating of plastic materials, such as PVC or polyurethane, such monomers can relatively easy penetrate into the plastic material in such a manner that they will not take part in the hardening polymerisation process or curing following the coating process. As a result, it is extremely difficult or even impossible in practise to avoid residues of non-reacted monomers in the obtained coated articles.

As mentioned, Ritter uses a coating solution which must contain the hydrophilic polymer or polymers ensuring the desired friction-reducing properties of the coating. It would, however, also be desirable to keep the coating fixed on the coated article to a sufficient extent even when the article is to stay for a long period in an aqeuous environment, such as within a body cavity. Thus it is usually desirable to use a binder having a sufficient affinity to the article to be coated. When it is a coating on an article being inserted for a long period of time in the human body, it is of vital importance that the affinity is sufficiently strong. In order to ensure a sufficiently strong affinity, it is usually necessary for the binder per se to be of a relatively strong, hydrophobic character. Accordingly, it is rather difficult to solve the above problem by using a coating material containing all the ingredients in dissolved condition as suggested by Ritter because a binder having a sufficiently good affinity to the article to be coated is rather difficult to dissolve in a solvent also suitable for dissolving the hydrophilic polymers, especially when it is desired to avoid the use of a monomeric starting material for the binder. In addition, should it in fact be possible to include both a hydrophilic polymer and a sufficiently efficient binder in the same solvent, then it is difficult to control the achieved structure of the binder and the hydrophilic polymer in the finished coating.

In connection with production of durable friction-reducing coatings it is a difficult compromise to provide both the desired hydrophility combined with a binding ensuring that the hydrophilic polymer is only slowly washed out at the same time as it is ensured that the coating adheres sufficiently to the coated article. The result depends in particular on the following factors:
- The weight ratio of hydrophilic polymer (b) to the hydrophobic binder (a).
- The molecular size of the hydrophilic polymer (b). It should be noted that even a relatively large molecular size of the hydrophilic polymer (b) does not eliminate a risk of a too quick washing out of the hydrophilic polymer.
- The degree of hydrophility/hydrophobicity of the binder (a). When the binder only possesses a reduced degree of hydrophobicity corresponding to a certain degree of hydrophility, an excellent affinity is indeed obtained between the binder (a) and the hydrophilic polymer (b), but at the expense of the affinity between the article to be coated and the binder (a).

Various solutions of the above problem have been suggested. The US-PS Nos. 4,487,808 and 4,666,437 (both Lambert) suggest application in sequence of two coatings in such a manner that initially a solution is applied of a compound with at least two non-reacted isocyanate groups per molecule followed by evaporation of the solvent. Subsequently, a solution of a hydrophilic polymer, polyethyleneoxide or PVP, respectively, is applied again followed by evaporation of the solvent, whereafter the material is cured at an increased temperature. By such a coating process the non-reacted isocyanate groups ensure a certain degree of adherence of the hydrophilic polymer. As a result, some of the groups providing the hydrophilic polymer with its hydrophilic properties are bound in such a manner that some reduction of the hydrophilic properties of the coating occurs. However, when this binding of the hydrophilic groups is restricted, it is on the other hand impossible to ensure that the hydrophilic polymer is not washed out too quickly.

An advantage would accordingly be found in a possibility of ensuring a suitable structure of the coating, where the binder (a) and the hydrophilic polymer (b) are dispersed in such a manner that both a good adherence to the coated article and a sufficient securing of the hydrophilic polymer (b) against an undesired quick washing out are obtained.

WO Publication No. 90/05162 (UNO PLAST A/S) discloses a method where attempts have been made at solving the above problem by applying a coating material containing a mixture of a hydrophobic polymer as binder in form of polyurethane without free isocyanate groups, i.e. substantially completely reacted polyurethane, and a hydrophilic polymer in form of PVP in at least two solvents of a different volatility. The two solvents present a solvent system of such a nature that the polyurethane is at least soluble both in the mixture of solvents and in the least volatile solvent of the two solvents, whereas PVP is soluble in the most volatile solvent of the two solvents and no more than partially soluble in the least volatile solvent of said solvents. This method allows a controlled precipitation of the two polymers, because PVP is initially precipitated, viz. when the most volatile solvent has evaporated with the effect that an improved dispersion of the two polymer ingredients is obtained. The method allows the use of a ratio of hydrophilic polymer (PVP) to binder of 1.5:1-5:1, preferably 2:1-3:1. In order to improve the hydrophilic character of the total coating an advantage would be found in a possibility of increasing the amount of the hydrophilic polymer.

US-PS No. 5,041,100 (Rowland et al.) discloses a hydrophilic friction-reducing coating to be applied to the outer surface of a catheter. The coating comprises an intimate mixture of polyurethane as a binder and high molecular weight polyethyleneoxide as a hydrophilic friction-reducing polymer. The coating is not to be further cured or hardened after the application on the catheter. According to the embodiments disclosed the ratio of the hydrophilic polymer to the binder is not greater than 0.125 parts by weight of the binder. By such ratio only a low friction-reducing effect can be obtained.

It has now been found that it is possible to provide a hydrophilic coating with a reduced friction in wet condition and a high amount of hydrophilic polymer. At the same time a good adherence of the coating is obtained by applying a coating material in form of a dispersion containing a radiation-cureable binder instead of applying hydrophilic polymer and binder either in form of two separate coating solutions, in form of a common solution or as a dispersion without a cureable binder,said dispersion being composed of a disperse phase dispersed in a dispersant, where the binder is a curable prepolymer and is contained in the disperse phase, whereas the hydrophilic polymer or a portion thereof is dissolved in the dispersant.

The present invention provides a method of producing an article with a coating having friction-reducing properties in wet condition and comprising a binder (a), a hydrophilic polymer (b), as well as, if desired, an osmolality-increasing compound (c), is thus characterised by producing a dispersion from
(a) a binder in form of a with radiation hardenable or curable urethane acrylate prepolymer of a molecular weight of at least 200,
(b) one or more hydrophilic polymers selected from polyvinyl pyrrolidone (PVP), polyethylene glycol (PEG), carboxymethylcellulose (CMC) and alginate,
(c) if desired, an osmolality-increasing compound, and
(d) a solvent or a mixture of solvents, in which (a) is substantially insoluble,
using 0.2 to 60 parts by weight of hydrophilic polymer (b) per part by weight of binder (a), applying said dispersion onto the article, removing the solvent or a portion thereof, and hardening the binder.

The method according to the invention provides essential advantages compared to the prior art disclosed by US-PS No. 5,005,287 (Ritter) and WO Publication No. 90/05162 (UNO PLAST A/S), because unlike these publications the method according to the invention does not use a solvent in which the vinyl monomer or the binder component, respectively, is soluble, but instead the method involves application of a dispersion. As a result, it is possible to use as dispersant a solvent in which only the hydrophilic polymer and the optional osmolality-increasing compound, such as sodium chloride, are soluble, i.e. it is usually possible to use water as dispersant and solvent. The use of organic solvents, such as methylene chloride, has thus been avoided, said methylene chloride having a tendency to penetrate into for instance a PVC article. Furthermore the methylene chloride is difficult to remove from the solids of the coating material and may accordingly remain as residues in the finished article. Solvents of a high affinity to the binder and the article per se are quickly absorbed therein and are thereby difficult to remove from the finished article even after sterilization of the latter. It is a well-known fact that such organic solvents are injurious to the human organism and therefore undesired as a general rule.

A further advantage is obtained by the use of a dispersion as coating material. The binder in such a coating material need not be present in a dissolved condition, which opens the possibility of using rather sparingly soluble prepolymers as binder. In this manner it is possible to avoid the use of monomers which may be injurious to health.

The use of a binder being hardened after the coating procedure renders it possible to adapt the position of the binder in the coating to each particular purpose. The binder is applied in form of the disperse phase in undissolved form, and after the hardening the structure thereof is accordingly deriving from the structure of the disperse phase. It is assumed that the disperse phase after the application forms a network of the original, substantially ball-shaped particles, such as drops, which after coalescing or compacting are kept together and to the article to be coated in such a manner that a structure is obtained which is in an excellent manner can retain the hydrophilic polymer and control the desired washing out thereof in the actual use situation for obtaining a lubricating effect for a long period.

It turned out that the method according to the invention renders it possible to obtain a durable coating excellently adhering to a resilient article, where the ratio of hydrophilic polymer to hydrophobic binder is up to about 29:1, cf. Example 1, i.e. substantially higher than the ratio obtainable according to the WO Publication No. 90/05162 (UNO PLAST A/S). The actual upper limit of the amount of hydrophilic polymer is usually determined by the increased tendency of the coating turning fragile or brittle. This tendency is particularly pronounced when the coated article is made of a resilient material. Thus it turned out to be possible to apply a durable coating onto a non-resilient material, where the ratio of hydrophilic polymer to hydrophobic binder is even higher, such as 55:1.

A high amount of hydrophilic polymer in the coating material presents, of course, an advantage when the desired lubricating effect is to be ensured in wet condition. In addition, a further advantage is obtained by keeping the amount of the hardenable or curable binder at a very low level because in this manner it is possible to use a radical-polymerisable binder of the type requiring the presence of a photoinitiator for the hardening. The low amount of binder means that it is possible merely to use a correspondingly low amount of photoinitiator considered to be injurious to health and irritating the mucosa. In addition, the photoinitiator can cause malodours.

The invention relates furthermore to a coating material comprising a binder (a), a hydrophilic polymer (b), as well as, if desired, an osmolality-increasing compound (c), said coating material being characterised in that it is provided in form of a dispersion having a disperse phase dispersed in a dispersant, said dispersion being produced from
(a) a binder in form of a radiation-curable urethane acrylate prepolymer of a molecular weight of at least 200,
(b) one or more hydrophilic polymers selected from PVP, PEG, CMC and alginate,
(c) if desired, an osmolality-increasing compound, and
(d) a solvent or a mixture of solvents, in which (a) is substantially insoluble,
and that it contains 0.2 to 60 parts by weight of hydrophilic polymer (b) per part by weight of binder (a).

According to an advantageous embodiment of the coating material the hydrophilic polymer ingredient (b) may be composed of
(b1) one or more hydrophilic polymers being only slightly soluble in the dispersant, and
(b2) one or more hydrophilic polymers being substantially completely soluble in the dispersant.

According to an additional, advantageous embodiment, the coating material may according to the invention comprise
3.5 to 15 parts by weight of the radation-curable urethane acrylate prepolymer (a),
60 to 240 parts by weight of the first hydrophilic polymer (b1),
50 to 200 parts by weight of the second hydrophilic polymer (b2),
22 to 90 parts by weight of osmolality-increasing compound (c), and
140 to 560 parts by weight of solvent (d).

An example of a hydrophilic polymer of the type (b1) is for instance polyethylene glycol (PEG), and an example of the hydrophilic polymer (b2) is for instance polyvinyl pyrrolidone (PVP), said hydrophilic polymers advantageously being used together with water as the solvent (d).

An advantageous radiation-curable urethane acrylate prepolymer (a) is found in one or more radical-polymerisable binders. When the coating material comprises a UV-curable or a laser beam-curable binder, said coating material comprises usually also a photoinitiator, i.e. preferably a photoinitiator conventional per se and capable of forming free radicals. The photoinitiator is advantageously of such a structure that each of the resulting free radicals includes an ethylenically unsaturation. In this manner it is ensured that the photoinitiator copolymerises with the binder with the effect that residues of non-reacted free radicals are avoided.

The extent of applicability of the invention appears from the following detailed description. It should, however, be understood that the detailed description and the specific examples are merely included to illustrate the preferred embodiments, and that various alterations and modifications within the scope of protection will be obvious to persons skilled in the art on the basis of the detailed description.

As mentioned, the method according to the invention involves the use of a coating material in form of a dispersion, usually an emulsion.

This dispersion can be produced by using a solvent, such as water, in which the binder used is insoluble.

It turned out that the use of a dispersion renders it possible to obtain an advantageous structure of the finished coating, when said dispersion includes a binder which is insoluble therein and thus is present in form of small ball-shaped particles forming the disperse phase. After the application, the ball-shaped particles present in the coating material coalesce when the solvent evaporates, whereafter it is possible to harden the binder while obtaining an advantageous coherent network structure.

Such a structure cannot be obtained when a dissolved binder (a) is used because the use of dissolved materials does not allow the same possibility of controlling the obtained structure conditioned by factors such as the conditions applying during the evaporation of the solvent and the solubility of the materials present in the coating.

Thus it appears that it is an important condition for the method according to the invention that a non-soluble, radiation-curable prepolymer binder (a) is used.

In principle it is thus possible with respect to the structure of the obtained coating to use any type of radiation-curable urethane acrylate prepolymer as the binder (a) provided said binder is insoluble in the solvent used.

When the article with a friction-reducing coating produced by the method according to the invention is an article to be used for insertion into a body cavity, such as for surgery, therapy or diagnostic use, particular requirements are, of course, presented to the coating material used, which must be neither toxic for nor irritating the mucosa. Most monomer materials, which are reactive by nature, are of a relatively low molecular weight, and have a recognized or assumed injurious effect to health. Bearing this in mind, one of the essential features of the present invention is that it does not use a monomer binder, but instead an oligomer prepolymer of a molecular weight of at least 200, preferably at least 300, and most preferred at least 400, such as in the range 700 to 5,000, especially 1,000 to 3,000. It is both quicker and easier to control the hardening of such prepolymers than the hardening by way of polymerisation based on monomers.

The use of prepolymers eliminates furthermore a tendency of the coating becoming sticky, which may apply to the use of monomers, especially when several different monomers are used.

Furthermore the use of a monomer binder causes the coating to shrink heavily during the polymerisation, which indeed provides a physical adhering effect. Such a coating fixed by way of shrinking may, however, loosen when immersed into water due to a poor chemical contact with the surface of the article. When an oligomer prepolymer is used instead, the shrinking effect is less pronounced. In addition, the chemical contact or binding to the surface of the article is efficient because the prepolymer can only penetrate a short distance into the surface of the article during the providing of a suitable binding. Unlike the prepolymer, the monomer often penetrates deeply into the surface of the article by diffusion and/or dissolving of said surface with the result that the monomer is lost for the polymerisation process and thus cannot contribute to the desired chemical binding.

In practise it turned out that coatings produced by the method according to the invention are fixed to the surface of the article and remain smooth even when immersed into water for 24 hours. These properties are also maintained when immersed into water for long periods of time. Thus long period tests revealed that a coated article produced according to Example 1 turned out to be durable, even after several days in the water. Such a feature is very important for catheters as it ensures a good sliding effect both when the catheter is to be inserted and when it is to be removed after insertion for a long period in the human body. Even the point of the catheter, which is particularly exposed to damages, preserves its smooth surface.

An advantageous type of the hardenable prepolymers is radiation-curable free-radical-polymerisable urethane acrylate prepolymers, such as electron radiation-curing, laser beam-curing or UV-curing prepolymers.

The use of UV-curing or laser beam-curing prepolymers usually requires the presence of a photoinitiator, which is a compound forming free radicals when subjected to ultraviolet light or laser beams. Such photoinitiators are well-known, and any type of photoinitiator having these properties can in principle be used. As free radicals may be injurious to health, it is, however, usually important to avoid residues of such radicals in the obtained coating. Avoidance of such residues can be ensured by using a photoinitiator comprising an ethylenically unsaturation in addition to the portion of the molecule being active at the formation of the free radicals, whereby the free radicals are copolymerised with the prepolymer during the polymerisation process. In this manner it is possible to efficiently avoid the presence of undesired, reactive low molecular weight compounds in the coating.

The technical development within the field of radiation-curing binders holds out a promise of useful alternative embodiments of the invention, where electron radiation is used instead of UV-curing or laser beam curing. These methods are expected to allow elimination of the use of photoinitiator.

An additional important feature of the binder (a) used is that it must have a sufficient affinity to the material of the article to be coated. A person skilled in the art can in a simple manner determine the affinity of a selected binder on the basis of solubility parameters of the material and the binder, respectively, or by way of simple immersion tests revealing whether the binder attacks the material and binds thereto.

The prepolymers of urethane acrylates are suitable by revealing a certain degree of hydrophility combined with a good affinity to for instance PVC, and which are particularly suited for resilient surfaces.

As the hydrophilic polymer(s), which are to ensure the sliding effect, i.e. the low friction of the coating in wet condition, PVP, PEG, CMC and alginate can be used.

The hydrophilic properties of the polymer constitute a condition that a polymer is able to provide a friction-reducing effect and thus can act as a sliding polymer. Thus a sliding effect can be obtained by means of a water-soluble polymer which can be released slowly in form of a viscous, lubricating solution. However, the sliding effect can also be obtained by means of a hydrophilic polymer, which is only slightly water-soluble, but which swells when contacted with water, since such a swollen water-containing polymer can provide a smooth surface too.

As mentioned above, the urethane acrylate prepolymers used as the binder (a) provide a hydrophility though said hydrophility is rather limited. The hydrophility can be determined in connection with a certain degree of swelling and water-binding after contact with water for a long period of time. As a result, the friction is indeed reduced though only slightly, but it provides an advantage if the actual hydrophilic slide polymer has been washed out too heavily from the article having been inserted into a body cavity for a long period of time.

Although the binder (a) thus may have a certain degree of hydrophility in some cases, the designations binder (a) and hydrophilic polymer (b), respectively, are, however, used in general in the present description and the claims in such a manner that these two types are distinguished from each other. Thus a binder (a) having a certain degree of hydrophility is not to be considered included by the general designation hydrophilic polymer (b).

The content of the hydrophilic polymer (b) is preferably at least 0.2, preferably at least 2.0, more preferred at least 6, especially at least 15, and most preferred at least 25 parts by weight per part by weight of binder (a). When the coating material is to be applied onto resilient articles, such as articles of PVC or polyurethane, the content of the hydrophilic polymer (b) is preferably max. 40, more preferred max. 35, and most preferred max. 31 parts by weight per part by weight of the binder (a). When the coating material is to be applied onto rigid articles, such as articles made of metal or glass, the content of the hydrophilic polymer (b) is preferably max. 60 and more preferred max 55 parts by weight per part by weight of the binder (a).

Thus polyvinyl pyrrolidone (PVP), polyethylene glycol or a combination thereof is usually, but not exclusively used as the hydrophilic polymer (b). It is also possible to use other hydrophilic polymers, such as the ones mentioned in the above paper by Y.L. Fan, such as CMC and alginate.

Suitable hydrophilic polymers (b) are often of a molecular weight in the range of 10,000 to 500,000, such as 20,000 to 400,000. As described below, it is often advantageous to use a combination of a low molecular weight hydrophilic polymer, such as a polymer of a molecular weight in the range of 10,000 to 60,000, and a high molecular weight hydrophilic polymer, such as a polymer of a molecular weight in the range of 100,000 to 500,000, such as 300,000 to 400,000.

The desired sliding effect is probably particularly achieved by way of a slow release of the sliding polymer. Experience has taught that the desired effect highly depends on the molecular size of the hydrophilic polymer, since molecules of a small size are washed out more quickly than high molecular weight polymers. On the other hand, the high molecular weight polymers provide no immediate sliding effect, whereas low molecular weight polymers provide such an immediate sliding effect. Accordingly, an advantage is found in combining sliding polymers of various molecular sizes for obtaining the desired sliding effect. When PVP is used as sliding polymer, the above advantage can be exemplified by a combination of PVP K30 (molecular weight about 40,000) and PVP K90 (molecular weight about 360,000). PVP K30 and K90 are standard products sold by several suppliers, such af BASF, Germany, and GAF CHEMICALS CORPORATION, Wayne, New Jersey, USA.

The tests performed so far have revealed that a mixture ratio of PVP K30 to PVP K90 of about 1:1, such as between 1:2 to 2:1, proved to be particularly advantageous. Based on these results, the person skilled in the art can easily find suitable combinations of hydrophilic polymers by means of PVP of other molecular weights or by means of other hydrophilic polymers in order to obtain a corresponding effect.

It is possible to use a particular type of hydrophilic polymer as a part of the hydrophilic polymers used, viz. a hydrophilic polymer of the type bl being only partially or sparingly soluble in the dispersant. This property is found for instance in polyethylene glycol (PEG) which is water-soluble, but precipitates like proteins in a concentrated solution of salt in water. Thus the use of sparingly soluble hydrophilic polymers renders it possible for this polymer or a portion thereof to enter the disperse phase of the coating material. As a result, a portion of the hydrophilic polymer ingredient can be intimately mixed with the binder, whereby an excellent affinity between the binder phase and the hydrophilic phase can be ensured in the coating, i.e. the portion of the dispersant remaining after the drying of the coating.

The hydrophilic polymer (b) can, as mentioned, be formed by a combination of one or more hydrophilic polymers being only sparingly soluble in the dispersant, here designated type b1, and one or more hydrophilic polymers being substantially completely soluble in said dispersant, here designated type b2. The hydrophilic polymer (b) can, however, also exclusively be one of these types, especially type b2 alone, as type b1 is often applicable only under particular conditions, inter alia depending on the degree of the solubility. In practise almost all weight ratios of b1 to b2 turned out to be applicable. Thus it is for instance possible to use 0.5 to 8 parts by weight of b1 per part by weight of b2, such as 1.0 to 3.0. Tests have also been carried out on 29 parts by weight of b1 per part by weight of b2.

During the use of the hitherto known hydrophilic coating materials for articles to be inserted into human body cavities, it turned out that it is often desirable to incorporate an osmolality-increasing compound, such as sodium chloride, in the coating in order to avoid a dehydration of the coating while said coating is in contact with body fluids. According to EP-PS No. 217,771 (Johansson et al.; Astra Meditec AB) it is suggested to treat the article with a solution of an osmolality-increasing compound, such as an aqueous salt solution, in an additional step following the application and the hardening of the hydrophilic coating. Such a further step is avoided by the method according to the invention, the osmolality-increasing compound optionally being present in the coating material in form of an ingredient of the dispersant phase.

The coating material includes, as mentioned above, usually also an osmolality-increasing compound, usually in form of sodium chloride. Based on tests carried out it must be assumed that this ingredient can have an essential effect on the structure of the coating material, at least in the embodiment also using a partially soluble hydrophilic polymer of the type (b1) as for instance PEG together with soluble hydrophilic polymers of the type (b2), such as PVP. These tests have indeed revealed that PEG is insoluble in a 25% sodium chloride solution whereas PVP is soluble in such a 25% sodium chloride solution. Thus it can be assumed that the presence of the osmolality-increasing compound (c) has an effect in ensuring the above described, particularly advantageous structure.

The most essential requirement presented to the used solvent (d) for achieving the above network-like structure of the obtained coating is that the binder (a) used is insoluble therein, i.e. in such a manner that it is ensured that the coating material forms a dispersion. Another vital requirement is that at least some of or all the hydrophilic polymers are soluble in the solvent (d).

In most cases it is preferred to use water as solvent (a) since water is inexpensive and non-toxic and furthermore meets the above requirements. It is, however, also possible to use other solvents meeting the above requirements, such as lower alcohols, such as ethanol and 2-propanol, and esters, such as ethylacetate, said solvents usually being mixed with water.

The amount of solvent (d) should usually be kept at a minimum because it is thereby ensured that the coating material achieves a suitably high viscosity. A high viscosity is of vital importance for allowing application of the coating of a suitable layer thickness, preferably in a single application step. The use of a too high amount of solvent has the effect that it is difficult to make the coating adhere to the article, said coating material then having a tendency to be repellant. In addition, a high amount of solvent requires much time and/or energy for the evaporation.

When the coating material has a tendency to be repellant, such as when the fluid coating material has a too high surface tension relative to the layer therebelow, it is, however, possible to solve the problem in a conventional manner by adding a surfactant.

Some application methods, such as spraying, often necessitates the use of a high amount of solvent since a low viscosity is desired.

An example of a coating material with a low amount of solvent (d) can be composed as follows:
3.5 to 15 parts by weight of radiation-curable urethane acrylate prepolymer (a),
100 to 450 parts by weight of hydrophilic polymer (b),
0 to 90 parts by weight of osmolality-increasing compound (c), and
140 to 560 parts by weight of solvent (d), or
3.5 to 15 parts by weight of radiation-curable urethane acrylate prepolymer (a),
0 to 140 parts by weight of the first hydrophilic polymer (b1),
50 to 450 parts by weight of the second hydrophilic polymer (b2),
0 to 90 parts by weight of osmolality-increasing compound (c), and
140 to 560 parts by weight of solvent (d).

The coating can be applied in a single step in any conventional manner. It is, however, also possible to apply the coating in several steps, especially when the coating material is of a low viscosity.

Beyond the indicated ingredients, the coating material according to the invention can include adjuvants and additives conventional to coating materials. However, it turned out surprisingly that it is possible to produce the coating material while achieving a dispersion by simply mixing the ingredients at a suitable temperature, such as heating to at least 60°C, without the use of a dispersing adjuvant, such as an emulgator. As a result, a particular advantage is obtained because conventional emulgators usually necessary by the production of dispersions may have a locally irritating effect.

It is of vital importance for the use of the coating materials according to the invention on articles to be in contact with the human body that it is possible to avoid ingredients of a low molecular weight apart from physiologically compatible ingredients, such as water and non-toxic osmolality-increasing compounds, such as sodium chloride.

The optionally present osmolality-increasing compound can be any compound providing a suitable compensation for the osmotic pressure difference between the moistured coating and the surrounding body fluids. Such compounds are water-soluble compounds of a low molecular weight, preferably such compounds that are dissociated in aqueous solution. Examples of such compounds are especially inorganic salts, such as sodium chloride, potassium chloride, potassium iodide, potassium nitrate, and calcium chloride, or organic salts, such as sodium citrate. Other water-soluble compounds can in principle be used too. Thus sodium benzoate, mono or disaccharides, such as glucose, and sugar alcohols, such as sorbitol, can be used too, all said osmolality-increasing compounds being mentioned in EP-PS No. 217,771 and the corresponding US-PS No. 4,906,237 (Johansson et al.).

In practise it turned out that the article coated by the method according to the invention can tolerate sterilisation without turning adhesive or sticky. The sterilisation is performed after packaging of the article and by way of treatment with water steam and ethyleneoxide at 60°C for a long period of time, such as over night, which is surprising as the person skilled in the art might expect the coating to be heavily adhesive under such conditions, said coating still containing a certain amount of water bond in the hydrophilic polymer after the hardening.

The binder used should preferably be a fluid binder in which case the dispersion is an emulsion. In this case, an advantageous coalescence of the binder is ensured prior to the hardening. The best results are obtained by means of a relatively viscous binder.

The hydrophilic polymer(s) used should be a combination of a low molecular weight hydrophilic polymer and a high molecular weight hydrophilic polymer, inter alia because the use of a high molecular weight hydrophilic polymer alone can face difficulties in obtaining a sufficiently thick coating, and because such a high molecular weight hydrophilic polymer has a tendency to turn adhesive or sticky when the amount of solvent is too low. These problems are solved by a low molecular weight hydrophilic polymer also being capable of acting as a kind of filler in addition to having an effect as slide polymer.

The method according to the invention provides a solution of the purpose aimed at which is particularly suitable for the environment because the use of solvents and monomers injurious to health is avoided. As a result, the disposal problems of waste substances injurious to health have been avoided at the same time as a health risk and malodours are avoided during the production. In addition residues injurous to health are avoided in the obtained product.

### Example 1

A coating mixture is produced from the following ingredients:

| | |
|---|---|
| Ebecryl 4830 | 7.5 g |
| Uvecryl P36 | 0.15 g |
| Polyethylene glycol 35,000 | 120.0 g |
| PVP K30 | 45.0 g |
| PVP K90 | 52.5 g |
| NaCl | 45.0 g |
| Water | 280.0 g |

Ebecryl 4830 is an acrylated urethane resin of a molecular weight of about 1,200, supplied by Radcure Specialties s.a., B-1620 Drogenbos, Belgium, in form of 90% polymer solid in tetraethylene glycoldiacrylate.

Uvecryl P36 is a copolymerisable photoinitiator in form of unsaturated copolymerisable benzophenon supplied by Radcure Specialties s.a.

PVP K30 is a polyvinyl pyrrolidone of a molecular weight of about 40,000, and PVP K90 is a polyvinyl pyrrolidone of a molecular weight of about 360,000.

The coating material is produced from the above ingredients by heating to 90°C by means of a magnetic stirrer. Ebecryl 4830 and Uvecryl P36 are, however, mixed with each other before they are mixed with the remaining ingredients.

A PVC tubing of a quality used for the production of catheters is coated with the mixture, whereafter the coating is dried and cured by means of ultraviolet light.

A dry, sterilisable coating is obtained, which in wet condition turns very smooth and adheres well to the PVC tubing used.

The weight ratio of hydrophilic polymers to the hydrophobic binder in the produced coating is 29:1.

The friction of the cured coating in wet condition is determined by a modification of the method according to ASTM D1894-90 using the test procedure shown in Fig. 1c therein. The test was carried out with a test block provided on the bottom side with two grooves. Two tubings produced according to Example 1 are placed in these two grooves. The test block ensures a vertical load of 5.970 N. The friction measurement is performed by means of a wet wash leather as base, and the test pulling is carried out in such a pulling direction that the test block slides like a sledge with the tubings as runners. The testing is carried out at a speed of 100 mm/min, and the test length is 25 mm. This test was carried out on four tubings all coated by the method according to Example 1, and it resulted in a kinetic friction factor of 0.172; 0.206; 0.198; and 0.171, respectively, without sterilisation. On three tubings prepared in the same way and then sterilised 0.169; 0.197; and 0.15, respectively, were measured.

### Example 2

The present Example illustrates the production of a coating material not containing sodium chloride.

A coating mixture is produced from the following ingredients:

| | |
|---|---|
| Ebecryl 4830 | 7.5 g |
| Uvecryl P36 | 0.15 g |
| Polyethylene glycol 35,000 | 120 g |
| PVP | 16 g |
| Water | 152 g |

The ingredients are mixed while being heated to about 90°C by means of a magnetic stirrer.

The weight ratio of binder to hydrophilic polymer was in this case 1:18.

The coating material was applied onto an article of plastic and an article of metal, respectively, and the resulting coating revaled in both cases a satisfying binding and sliding effect.

### Example 3

The present Example illustrates the production of a coating material exclusively comprising hydrophilic polymer of the type b2.

A coating mixture was produced from the following ingredients:

| | |
|---|---|
| Ebecryl 4830 | 7.5 g |
| Uvecryl P36 | 0.15 g |
| PVP 30 | 45 g |
| PVP 90 | 52,5 g |
| Sodium chloride | 21 g |
| Water | 209 g |

The ingredients are mixed while being heated to 90°C by means of a magnetic stirrer.

The weight ratio of binder to hydrophilic polymer was in this case 1:13.

The coating material was applied onto an article of plastic and an article of metal, respectively, and the resulting coating revaled in

both cases a good adherence to the article, a satisfying curing, and a satisfying sliding effect.

### Example 4

Like in the above Examples, a satisfying coating material was produced from the following ingredients and comprising hydrophilic polymers both of the type b1 and the type b2:

| | |
|---|---|
| Ebecryl 4830 | 7.5 g |
| Uvecryl P36 | 0.15 g |
| Polyethylene glycol 35,000 | 217.5 g |
| PVP 90 | 7.5 g |
| Sodium chloride | 45 g |
| Water | 135 g |

The weight ratio of binder to hydrophilic polymer was in this case 1:30.

### Example 5

The present Example illustrates the production of a coating material exclusively comprising hydrophilic polymers of the type b2:

| | |
|---|---|
| Ebecryl 4830 | 7.5 g |
| Uvecryl P36 | 0.15 g |
| PVP 30 | 100.4 g |
| PVP 90 | 117.1 g |
| Sodium chloride | 45 g |
| Water | 460 g |

The ingredients are mixed while being heated to about 90°C by means of a magnetic stirrer.

The weight ratio of binder to hydrophilic polymer was in this case 1:29.

The coating material was applied onto a PVC tubing, whereafter the coating was dried and cured by means of ultraviolet light.

The resulting coating revealed a good adherence to the PVC tubing and an excelling sliding effect.

### Example 6

The present Example illustrates the production of a coating material with a high amount of hydrophilic polymer, said coating material being suited for coating of metal and glass surfaces.

A coating mixture was produced from the following ingredients:

| | |
|---|---|
| Ebecryl 4830 | 7.5 g |
| Uvecryl P36 | 0.15 g |
| Polyethylene glycol 35,000 | 227.7 g |
| PVP 30 | 82.9 g |
| PVP 90 | 101.9 g |
| Sodium chloride | 84 g |
| Water | 529 g |

The weight ratio of binder to hydrophilic polymer was in this case 1:55.

The coating material was applied onto a surface of glass and a surface of metal, respectively.

After the drying and the curing of the coating by means of ultraviolet light, a very satisfying coating was obtained adhering well both to the glass and to the metal surface.

### Example 7

A mixture with a ratio of hydrophobic binder polymer to hydrophilic polymer of 1:1.0 was made according to the formulation in Table 1. This composition was applied to a plasticized PVC catheter and cured with UV radiation as in the previous Examples. The coated catheter was then immersed in water for 10 sec. A significant frictional reduction compared with the uncoated catheter was obtained. There was a rapid release of water-soluble friction-reducing polymer which was easily discerned by gently rubbing the wetted catheter with two fingers. The release of hydrophilic polymer is also readily confirmed by subsequently rubbing the fingers against each other. Adhesion to the catheter was excellent.

### Example 8

A mixture with a ratio of hydrophobic polymer to hydrophilic polymer of 1:0.5 was made with the formulation given in Table I. The curing and testing procedures described in the previous Example confirmed that the release of hydrophilic polymer and frictional reduction effect was also found in this case.

### Example 9

A mixture with a ratio of hydrophobic polymer to hydrophilic polymer of 1:0.1 was made with the formula given in Table I. The same curing and testing procedures as described in the previous Examples was done, however, in this case there was no significant frictional reduction or release of hydrophilic polymer.

**Table I**

| EXAMPLE | 7 | 8 | 9 |
|---|---|---|---|
| Mixture ratio Binder polymer:hydrophilic polymer | 1:1 | 1:0.5 | 1:0.1 |

| Content | Amount g | Amount g | Amount g |
|---|---|---|---|
| Ebecryl 4830 | 108.75 | 217.50 | 543.75 |
| Uvecryl P36 | 2.18 | 4.35 | 10.88 |
| Polyethylene glycol 35,000 | 56.25 | 56.25 | 28.13 |
| PVP 30 | 22.50 | 22.50 | 11.25 |
| PVP 90 | 30.00 | 30.00 | 15.00 |
| Sodium chloride | 21.75 | 32.60 | 58.10 |
| Water | 165.75 | 176.55 | 217.63 |

It is obvious from the above description of the invention that it can be varied in many ways. Such variations are not to be considered deviations from the scope and idea of the invention, and all such modifications obvious to persons skilled in the art are also to be considered comprised by the following claims.

## Claims

1. A method of producing an article with a coating having friction-reducing properties in wet condition, said coating comprising a binder (a), a hydrophilic polymer (b), which is different from the binder (a), as well as, if desired, an osmolality-increasing compound (c), characterised by producing a dispersion from
(a) a binder in form of a radiation-curable urethane acrylate prepolymer of a molecular weight of at least 200,
(b) one or more hydrophilic polymers from the group of polyvinyl pyrrolidone (PVP), polyethylene glycol (PEG), carboxymethylcellulose (CMC) and alginate,
(c) if desired, an osmolality-increasing compound, and
(d) a solvent or a mixture of solvents, in which (a) is substantially insoluble,
using 0.2 to 60 parts by weight of hydrophilic polymer (b) per part by weight of binder (a), applying said dispersion onto the article, removing the solvent or a portion thereof, and hardening the binder.

2. A coating material comprising a binder (a), a hydrophilic polymer (b), which is different from the binder (a), as well as, if desired, an osmolality-increasing compound (c), characterised in that it is provided in form of a dispersion having a disperse phase dispersed in a dispersant, said dispersion being produced from
(a) a binder in form of a radiation-curable urethane acrylate prepolymer of a molecular weight of at least 200,
(b) one or more hydrophilic polymers from the group of polyvinyl pyrrolidone (PVP), polyethylene glycol (PEG), carboxymethylcellulose (CMC) and alginate,
(c) if desired, an osmolality-increasing compound, and
(d) a solvent or a mixture of solvents, in which (a) is substantially insoluble,
and that it contains 0.2 to 60 parts by weight of hydrophilic polymer (b) per part by weight of binder (a).

3. A coating material as claimed in claim 2, characterised by the hydrophilic polymer component (b) being composed of
(b1) one or more hydrophilic polymers being only slightly soluble in the dispersant, and
(b2) one or more hydrophilic polymers being substantially completely soluble in the dispersant.

4. A coating material as claimed in claim 3, characterised by comprising
3.5 to 15 parts by weight of radiation-curable urethane acrylate prepolymer (a),
60 to 240 parts by weight of the first hydrophilic polymer (b1),
50 to 200 parts by weight of the second hydrophilic polymer (b2),
22 to 90 parts by weight of osmolality-increasing compound (c), and
140 to 560 parts by weight of solvent (d).

5. A coating material as claimed in claim 3, characterised by the hydrophilic polymer (b1) being polyethylene glycol (PEG), by the hydrophilic polymer (b2) being polyvinyl pyrrolidone (PVP), and by the solvent (d) being water.

6. A coating material as claimed in claim 2, characterised by the binder (a) being one or more radical-polymerisable binders.

7. A coating material as claimed in claim 2, characterised by the binder (a) being a UV-curable or laser beam-curable binder, and by the coating material further containing a photoinitiator.

8. A coating material as claimed in claim 7, characterised by the photoinitiator being conventional per se with a capacity for forming free radicals, said photoinitiator being of such a structure that each of the resulting free radicals contains an ethylenically unsaturation.

9. An article with a coating having friction-reducing properties in wet condition obtainable by the method as claimed in claim 1.

## Patentansprüche

1. Verfahren zur Herstellung eines Artikels mit einer Beschichtung mit reibungsvermindernden Eigenschaften bei nasser Bedingung, wobei die Beschichtung ein Bindemittel (a), ein hydrophiles Polymer (b), das vom Bindemittel (a) verschieden ist, ebenso wie, falls gewünscht, eine Verbindung (c) zur Erhöhung der Osmolarität, umfaßt,
**gekennzeichnet durch**
die Herstellung einer Dispersion aus
(a) einem Bindemittel in Form eines strahlungshärtbaren Urethanacrylat-Prepolymer mit einem Molekulargewicht von wenigstens 200,
(b) einem oder mehreren hydrophilen Polieren aus der Gruppe Polvvinylpyrrolidon (PVP), Polyethylenglycol (PEG), Carboxymethylcellulose (CMC) und Alginat,
(c) falls gewünscht, einer Verbindung zur Erhöhung der Osmolarität, und
(d) einem Lösungsmittel oder einer Mischung aus Lösungsmitteln, in welchem (a) weitgehend unlöslich ist,
unter Verwendung von 0,2 bis 60 Gew.-Teilen eines hydrophilen Polymers (b) pro Gew.-Teil Bindemittel (a), und Anwenden der Dispersion auf den Artikel, Entfernen des Lösungsmittels oder eines Teils davon und Härten des Bindemittels.

2. Beschichtungsmaterial, umfassend ein Bindemittel (a), ein hydrophiles Polymer (b), das vom Bindemittel (a) verschieden ist, ebenso wie, falls gewünscht, eine Verbindung (c) zur Erhöhung der Osmolarität,
**dadurch gekennzeichnet,**
daß es in Form einer Dispersion mit einer in einem Dispergiermittel dispergierten dispersen Phase bereitgestellt ist, wobei die Dispersion hergestellt ist aus
(a) einem Bindemittel in Form eines strahlungshärtbaren Urethanacrylat-Prepolymer mit einem Molekulargewicht von wenigstens 200,
(b) einem oder mehreren hydrophilen Polymeren aus der Gruppe Polyvinylpyrrolidon (PVP), Polyethylenglycol (PEG), Carboxymethylcellulose (CMC) und Alginat,
(c) einer Verbindung zur Erhöhung der Osmolarität, falls gewünscht,
(d) einem Lösungsmittel oder einer Mischung aus Lösungsmitteln, worin (a) weitgehend unlöslich ist.
und daß es 0,2 bis 60 Gew.-Teile hydrophiles Polymer (b) pro Gew.-Teil Bindemittel (a) enthält.

3. Beschichtungsmaterial nach Anspruch 2,
**dadurch gekennzeichnet,**
daß die hydrophile Polymerkomponente (b) zusammengesetzt ist aus
(b1) einem oder mehreren hydrophilen Polymeren, die nur leicht löslich im Dispergiermittel sind, und
(b2) einem oder mehreren hydrophilen Polymeren, die weitgehend vollständig im Dispergiermittel löslich sind.

4. Beschichtungsmaterial nach Anspruch 3,
**dadurch gekennzeichnet,**
daß es umfaßt:
3,5 bis 15 Gew.-Teile eines strahlungshärtbaren Urethanacrylat-Prepolymer (a),
60 bis 240 Gew.-Teile des ersten hydrophilen Polymers (b1),
50 bis 200 Gew.-Teile des zweiten hydrophilen Polymers (b2),
22 bis 90 Gew.-Teile der Verbindung (c) zur Erhöhung der Osmolarität, und
140 bis 560 Gew.-Teile Lösungsmittel (d).

5. Beschichtungsmaterial nach Anspruch 3,
**dadurch gekennzeichnet,**
daß das hydrophile Polymer (b1) Polyethylenglycol (PEG) ist, das hydrophile Polymer (b2) Polyvinylpyrrolydon (PVP) ist und das Lösungsmittel (d) Wasser ist.

6. Beschichtungsmaterial nach Anspruch 2,
**dadurch gekennzeichnet,**
daß das Bindemittel (a) aus einem oder mehreren Radikalpolymerisierbaren Bindemitteln besteht.

7. Beschichtungsmaterial nach Anspruch 2,
**dadurch gekennzeichnet,**
daß das Bindemittel (a) ein UV-härtbares oder Laserstrahlhärtbares Bindemittel ist und daß Beschichtungsmaterial weiterhin einen Photoinitiator enthält.

8. Beschichtungsmaterial nach Anspruch 7,
**dadurch gekennzeichnet,**
daß der Photoinitiator ein konventioneller ist, der per se mit einer Kapazität zur Bildung freier Radikale ausgerüstet ist und der Photoinitiator solch eine Struktur besitzt, daß jedes der resultierenden freien Radikale eine ethylenische Ungesättigkeit enthält.

9. Artikel mit einer Beschichtung mit reibungsvermindernden Eigenschaften, bei nasser Bedingung, erhältlich durch das Verfahren nach Anspruch 1.

## Revendications

1. Procédé de production d'un article avec un revêtement ayant des propriétés diminuant le frottement en situation humide, ledit revêtement comprenant un liant (a), un polymère hydrophile (b), qui est différent du liant (a), ainsi que, le cas échéant, un composé augmentant l'osmolalité (c), caractérisé par la production d'une dispersion à partir de
(a) un liant sous forme de prépolymère d'uréthane-acrylate durcissable par les radiations d'un poids moléculaire d'au moins 200,
(b) un ou plusieurs polymères hydrophiles choisis dans le groupe de la polyvinylpyrrolidone (PVP), du polyéthylèneglycol (PEG), de la carboxyméthylcellulose (CMC) et de l'alginate,
(c) le cas échéant, un composé augmentant l'osmolalité, et
(d) un solvant ou un mélange de solvants, dans lequel (a) est pratiquement insoluble,
l'utilisation de 0,2 à 60 parties en poids de polymère hydrophile (b) par partie en poids de liant (a), l'application de ladite dispersion sur l'article, l'élimination du solvant ou d'une partie de celui-ci, et le durcissement du liant.

2. Matériau de revêtement comprenant un liant (a), un polymère hydrophile (b), qui est différent du liant (a), ainsi que, le cas échéant, un composé augmentant l'osmolalité (c), caractérisé en ce qu'il est fourni sous la forme d'une dispersion ayant une phase dispersée dispersée dans un dispersant, ladite dispersion étant produite à partir de
(e) un liant sous forme de prépolymère d'uréthane-acrylate durcissable par les radiations d'un poids moléculaire d'au moins 200,
(f) un ou plusieurs polymères hydrophiles choisis dans le groupe de la polyvinylpyrrolidone (PVP), du polyéthylèneglycol (PEG), de la carboxyméthylcellulose (CMC) et de l'alginate,
(g) le cas échéant, un composé augmentant l'osmolalité, et
(h) un solvant ou un mélange de solvants, dans lequel (a) est pratiquement insoluble,
et en ce qu'il contient 0,2 à 60 parties en poids de polymère hydrophile (b) par partie en poids de liant (a).

3. Matériau de revêtement selon la revendication 2, caractérisé en ce que le composant polymère hydrophile (b) est composé de
(b1) un ou plusieurs polymères hydrophiles qui ne sont que légèrement solubles dans le dispersant, et
(b2) un ou plusieurs polymères hydrophiles qui sont dans une large mesure complètement solubles dans le dispersant.

4. Matériau de revêtement selon la revendication 3, caractérisé en ce qu'il comprend
3,5 à 15 parties en poids de prépolymère d'uréthane-acrylate durcissable par les radiations (a),
60 à 240 parties en poids du premier polymère hydrophile (b1),
50 à 200 parties en poids du deuxième polymère hydrophile (b2),
22 à 90 parties en poids d'un composé augmentant l'osmolalité (c), et
140 à 560 parties en poids de solvant (d).

5. Matériau de revêtement selon la revendication 3, caractérisé en ce que le polymère hydrophile (b1) est le polyéthylèneglycol (PEG), en ce que le polymère hydrophile (b2) est la polyvinylpyrrolidone (PVP), et en ce que le solvant (d) est l'eau.

6. Matériau de revêtement selon la revendication 2, caractérisé en ce que le liant (a) consiste en un ou plusieurs liants polymérisables par les radicaux.

7. Matériau de revêtement selon la revendication 2, caractérisé en ce que le liant (a) est un liant durcissable par les UV ou durcissable par les faisceaux laser, et en ce que le matériau de revêtement contient en outre un photoinitiateur.

8. Matériau de revêtement selon la revendication 7, caractérisé en ce que le photoinitiateur est classique en soi avec la capacité de former des radicaux libres, ledit photoinitiateur ayant une structure telle que chacun des radicaux libres résultants contient une insaturation éthylénique.

9. Article avec un revêtement ayant des propriétés diminuant le frottement en situation humide, pouvant être obtenu par le procédé selon la revendication 1.
